# FASCICULE DE BREVET EUROPEEN

(11) **EP 2 094 631 B1**
(45) Date de publication et mention de la délivrance du brevet: **16.09.2015**
(21) Numéro de dépôt: 07871812.9
(22) Date de dépôt: 07.12.2007
(51) Int. Cl.: C07C 41/09, C07C 43/04

(54) **DESHYDRATATION DU METHANOL EN DIMETHYL ETHER EMPLOYANT DES CATALYSEURS A BASE D'UNE ZEOLITHE SUPPORTEE SUR DU CARBURE DE SILICIUM**
DEHYDRIERUNG VON METHANOL ZU DIMETHYLETHER ÜBER KATALYSATOREN MIT EINEM ZEOLITH AUF EINEM SILICIUMCARBID-SUBSTRAT
DEHYDRATION OF METHANOL INTO DIMETHYL ETHER USING CATALYSTS CONTAINING A ZEOLITHE ON A SILICON CARBIDE SUBSTRATE

(30) Priorité: 08.12.2006 FR 0610743
(43) Date de publication de la demande: 02.09.2009
(73) Titulaire: Centre National de la Recherche Scientifique (CNRS), 75016 Paris (FR); Université Louis Pasteur, U.L.P., 67000 Strasbourg (FR)
(72) Inventeur: IVANOVA, Svetlana, F-67000 Strasbourg (FR); VANHAECKE, Estelle, F-67000 Strasbourg (FR); LIBS, Suzanne, F-67300 Schiltigheim (FR); LOUIS, Benoit, F-67201 Eckbolsheim (FR); PHAM-HUU, Cuong, F-67700 Saverne (FR); LEDOUX, Marc, F-67000 Strasbourg (FR)
(74) Mandataire: Jacobson, Claude
(86) Numéro de dépôt international: PCT/FR2007/002017
(87) Numéro de publication internationale: WO 2008/090268

(56) Documents cités:
- WO-A-03/059509
- KI-WON JUN ET AL: "HIGHLY WATER-ENHANCED H-ZSM-5 CATALYSTS FOR DEHYDRATION OF METHANOL TO DIMETHYL ETHER" BULLETIN OF THE KOREAN CHEMICAL SOCIETY, KOREAN CHEMICAL SOCIETY, SEOUL, KR, vol. 24, no. 1, 2003, pages 106-108, XP009081850 ISSN: 0253-2964 cité dans la demande

## Description

La présente invention a trait au domaine de la conversion du méthanol en diméthyl éther. Plus précisément, l'invention concerne une méthode améliorée de conversion de méthanol en diméthyl éther particulièrement efficace qui s'avère adaptée en particulier à la production de diméthyl éther à grande échelle.

Le diméthyl éther (ou éther diméthylique), de formule CH₃-O-CH₃, est un composé dont la synthèse industrielle trouve de multiples applications.

En effet, le diméthyl éther est notamment utile à titre de précurseur pour la synthèse de divers composés d'intérêt comme des oléfines de bas poids moléculaire, l'acétate de méthyle ou bien encore le diméthyl sulfate. Il est également exploitable en tant que tel, par exemple à titre de gaz propulseur.

Plus spécifiquement, le diméthyl éther a été proposé à titre de carburant alternatif, pour remplacer avantageusement les dérivés du pétrole. Il s'agit en effet d'un gaz facilement liquéfiable (son point d'ébullition est de -25°C) qui a un indice de cétane comparable à celui du diesel. En outre, le diméthyl éther présente l'avantage d'être beaucoup moins polluant que les dérivés du pétrole, notamment en termes d'émission d'oxydes de soufre, d'oxyde d'azote et de suies, ce qui le rend plus compatible avec l'évolution récente des législations concernant les gaz d'échappement. Pour plus de détails concernant les avantages de l'utilisation du diméthyl éther à titre de carburant alternatif, on pourra notamment se reporter à l'article de Semelsberger et al. dans le Journal of Power Ressources vol. 152(1), pp. 87-89 (2005).

A l'heure actuelle, le diméthyl éther est déjà utilisé dans le secteur des fuels domestiques pour remplacer les gaz liquéfiés obtenus à partir du pétrole (butane et propane), et il est envisagé à titre de combustible pour produire de l'électricité à grande échelle, notamment en Inde où il devrait assurer à lui seul la moitié de la production d'électricité en 2010.

Une technique de production classique consiste à synthétiser le diméthyl éther à partir d'un mélange de CO et H₂ (mélange dit "gaz de synthèse") que l'on fait réagir sur un catalyseur adapté (à base d'oxyde métallique en général), par exemple selon les méthodes décrites par T. Ogawa et al. dans le Journal of Natural Gas Chemistry vol. 12, pp. 219-227 (2003), ou bien encore dans les documents GB 1 398 696, US 4,177,167, GB 2 099 327 ou GB 2 093 365.

Une autre technique de synthèse du diméthyl éther, développée plus récemment, consiste à préparer ce composé par déshydratation du méthanol sur un catalyseur acide, selon la réaction bilan suivante :

2 CH₃OH → CH₃-O-CH₃ + H₂O

Cette réaction a notamment été décrite par K.W. Jun et al. dans le Bulletin Korean Chemical Society vol. 24, p. 106 (2003).

La réaction de déshydratation du méthanol précitée met classiquement en oeuvre des catalyseurs solides à base d'alumine gamma ou d'alumine gamma modifiée, du type de ceux décrits par exemple dans les documents US 4,560,807, EP 270 852 ou GB 403 402. De tels catalyseurs présentent un inconvénient, à savoir que, compte tenu de leur caractère hydrophile, ils se désactivent en présence d'eau, ce qui interdit notamment leur utilisation pour la conversion du méthanol issu de la biomasse, sauf à mettre en oeuvre des procédés lourds et onéreux de pré-traitement du méthanol.

Pour pallier les inconvénients rencontrés avec les catalyseurs de type alumine gamma, il a été proposé des systèmes catalytiques plus particuliers.

Dans WO 04/74228, il est décrit un système dual de catalyseurs, utilisant un catalyseur acide hydrophile assurant la conversion du méthanol en diméthyl éther couplé à un catalyseur acide hydrophobe assurant le maintien du méthanol dans un état de déshydratation. Ce système présente certes de bons rendements de synthèse, mais il s'avère quelque peu compliqué à mettre en oeuvre.

Pour catalyser la réaction de déshydratation du méthanol, il a également été envisagé l'emploi de zéolithes, notamment des zéolithes de type MFI, telles que, par exemple, la zéolithe ZSM-5, qui sont relativement stables en présence d'eau, et dont l'acidité peut être modulée, notamment en les imprégnant par une solution de sels de sodium, ce qui permet d'obtenir de relativement bons rendements.

Néanmoins, en marges de ces différents avantages, l'emploi de zéolithes du type de la zéolithe ZSM-5 ne s'avère pas entièrement satisfaisant en pratique, et ce en particulier lorsqu'on souhaite mettre en oeuvre la réaction de déshydratation à grande échelle.

En effet, il est à souligner que ces catalyseurs présentent un inconvénient majeur, à savoir qu'ils ne sont pas stables au cours du temps. Plus précisément, il s'avère que, lorsqu'on emploie des catalyseurs constitués de zéolithe de type ZSM-5 pour effectuer la réaction de conversion du méthanol en diméthyl éther, on observe en général une perte très rapide de l'activité du catalyseur, qui se traduit concrètement par une diminution très nette de la conversion du méthanol au cours du temps, cette perte d'activité étant généralement observée après des temps de réaction très courts, tout au plus de l'ordre de quelques heures, typiquement, après 2 à 6 heures de réaction.

Il semble pouvoir être avancé que cette perte d'activité catalytique observée lorsqu'on utilise des catalyseurs constitués de zéolithe telle que la ZSM-5 s'explique au moins en partie un phénomène dit de cokage, à savoir le dépôt progressif de carbone au sein de la structure de la zéolithe. Sont également à prendre en compte une altération possible de la structure zéolithique par la vapeur d'eau formée au cours de la réaction de conversion du méthanol en diméthyl éther, ainsi que l'influence potentielle de la régénération sous air de la structure du catalyseur. De plus, la réaction de conversion est exothermique ce qui est susceptible de former des points chauds sur le catalyseur, propres à promouvoir les phénomènes précités.

En outre, les catalyseurs constitués de zéolithes telles que la ZSM-5 présentent un autre inconvénient : ils se présentent le plus souvent sous forme de poudres d'emploi relativement difficile et qui conduisent notamment à des pertes de charge importantes au sein des réacteurs, ce qui empêche leur mise en oeuvre à une échelle industrielle. Pour éviter ce problème, il a été envisagé de mettre en forme les poudres de zéolithes sous forme de solides macroscopiques, notamment par extrusion en présence de liants inorganiques de type oxyde de silicium ou oxyde d'aluminium. Toutefois, cette solution ne s'avère pas satisfaisante notamment dans la mesure où les agents liants utilisés rendent une partie de la zéolithe inaccessible et ils sont en outre susceptibles d'induire des réactions secondaires indésirables.

Un but de la présente invention est de fournir une nouvelle méthode de synthèse du diméthyl éther par déshydratation du méthanol, qui soit adaptée à la production de diméthyl éther à grande échelle, et qui s'affranchisse des problèmes précités, en particulier en termes d'instabilité du catalyseur.

L'invention a pour objet un procédé de préparation de diméthyl éther par déshydratation catalytique du méthanol, dans lequel on emploie, à titre de catalyseur, une zéolithe H-ZSM-5 immobilisée sur un support de carbure de silicium β-SiC, où le support de carbure de silicium a une surface spécifique supérieure ou égale à 5 m²/g.

Les zéolithes selon l'invention sont mises en oeuvre à l'état supporté sur un support de carbure de silicium présentant une surface spécifique supérieure ou égale à 5 m²/g, par exemple supérieure à 10m²/g.

Par *"support de carbure de silicium",* on entend, au sens de la présente description, un support solide comprenant du SiC à titre de constituant majoritaire et qui est propre à immobiliser sur sa surface une couche de zéolithe.

En général, le support de carbure de silicium présent dans les catalyseurs de l'invention peut être exclusivementconstitué de SiC. Il n'est toutefois pas exclu que ce support contienne d'autres espèces que le SiC, notamment des impuretés inévitables.

Le carbure de silicium dans le contexte de la présente invention est restreint au carbure de silicium de structure bêta.

Le support de carbure de silicium utilisé est le plus souvent sous forme d'objets solides macroscopique, ayant des dimensions typiquement supérieures à 1 mm. Ainsi, il peut s'agir de grains, extrudés, bâtonnets, monolithes, tubes, trilobes ou anneaux ou bien encore des mousses, notamment des mousses alvéolaires rigides.

Au cours des travaux qui ont conduit à la présente invention, les inventeurs ont mis en évidence que l'immobilisation de zéolithes sur un support spécifique de carbure de silicium conduit à un effet tout à fait inattendu, à savoir une très nette stabilisation du catalyseur dans la réaction de conversion du méthanol en diméthyl éther, et ce tout particulièrement lorsque le support de carbure de calcium présente une surface spécifique élevée supérieure ou égale à 5 m²/g.

Plus précisément, alors que des zéolithes non supportées voient leur activité décroître rapidement au cours du temps, il s'avère que l'emploi selon l'invention d'une zéolithe déposée sur un support de carbure de silicium conduit, au contraire, à un maintien d'une activité catalytique sensiblement constante sur de très longues périodes, typiquement pendant au moins 20 heures, voire beaucoup plus.

La stabilisation obtenue dans le cadre de la présente invention semble s'expliquer, entre autres, par la très bonne conductivité thermique du support de carbure de silicium employé, qui permet d'évacuer très rapidement la chaleur formée au cours de la réaction de conversion du méthanol en diméthyl éther, évitant ainsi la formation de point chauds au sein du lit catalytique, ce qui réduit notamment les risques d'empoisonnement du catalyseur par cokage.

De plus, les zéolithes supportées sur SiC utiles selon la présente invention présentent une activité catalytique particulièrement élevée dans la réaction de conversion du méthanol en diméthyl éther, qui s'avère le plus souvent au moins comparable à celle des catalyseurs commerciaux usuels. Ainsi, en règle générale, le gain obtenu en termes de stabilité n'a pas de contrepartie sur l'efficacité du catalyseur. En d'autres termes, l'amélioration apportée dans le cadre de l'invention contribue effectivement à une amélioration globale des propriétés catalytiques.

En outre, la mise en oeuvre de zéolithes sous forme supportée sur un support de carbure de silicium conduit à des pertes de charges très faibles, qui sont en tout état de cause bien plus réduites que dans le cas de l'utilisation de zéolithes non supportées.

Un autre avantage des catalyseurs employés selon l'invention est que l'immobilisation des zéolithes sur un support de carbure de silicium conduit à une amélioration des transferts de masse et de chaleur dans le réacteur où est conduite la réaction de conversion du méthanol en diméthyl éther.

Par ailleurs, il est à noter que le support de SiC utilisé selon l'invention est un support rigide, qui confère de ce fait au catalyseur une bonne tenue mécanique. Le SiC est en outre d'un matériau inerte chimiquement, qui n'est donc pas susceptible de conduire à des réactions parasites lors de la réaction de conversion du méthanol en diméthyl éther.

Ces différents avantages font du procédé de la présente invention une alternative extrêmement intéressante aux méthodes de conversion du méthanol en diméthyl éther qui sont actuellement connues, et ce d'autant plus que l'emploi du catalyseur supporté de l'invention autorise l'utilisation de méthanol brut à forte teneur en eau (par exemple comprenant de 20 à 40% en volume d'eau), tel que le méthanol issu de la biomasse, qui est envisagé à l'heure actuelle à titre de matière première pour la fabrication de diméthyl éther à grand échelle à titre de carburant alternatif.

A titre de catalyseur à base de zéolithe utilisable dans le cadre de la présente invention on peut notamment mettre en oeuvre les catalyseurs d'alkylation et d'acylation des aromatiques décrits dans la demande de brevet WO 03/59509.

Plus généralement, le catalyseur utilisé selon l'invention peut notamment être synthétisé en effectuant le dépôt zéolithe sur un support de SiC par voie hydrothermale, selon des techniques connues en soi, notamment du type de celles décrites dans la demande WO 03/59509 précitée.

Selon un mode de préparation avantageux, le catalyseur utilisé selon l'invention est un catalyseur synthétisé en mettant en oeuvre les étapes suivantes :
(A) un traitement thermique (dit de calcination) du support de SiC, ce traitement étant généralement réalisé à une température de 800 à 1000°C (typiquement à une température de l'ordre 900°C) pendant quelques heures (le plus souvent de l'ordre de 2 à 6 heures), ce qui conduit à la formation d'une couche superficielle de silice sur la surface du support.
   Le traitement thermique ainsi opéré est en fait une étape de prétraitement du support qui induit une transformation des espèces carbure de silicium et oxycarbures de silicium de surface sous la forme de silice. La couche de silice obtenue sert de point d'ancrage pour le dépôt ultérieur de la zéolithe sur le support.
   Notamment de façon à assurer un ancrage efficace entre le support et la zéolithe déposée, il est préférable que la couche de silice obtenue en surface du support à l'issue de la calcination ait une épaisseur d'au moins 1 nm, et plus préférentiellement d'au moins 2 nm. Toutefois, il est préférable que l'épaisseur de la couche de silice réalisée reste suffisamment fine : trop épaisse, elle tend à se dissoudre lors du dépôt ultérieur de la zéolithe. Par ailleurs, le dépôt d'une couche de zéolithe suffisamment fine permet d'optimiser l'accessibilité du méthanol sur les sites réactifs ainsi que l'évacuation du diméthyl éther formé. En général, il est intéressant que la couche formée ait une épaisseur inférieure ou égale à 10 nm. Avantageusement, la couche de silice formée a une épaisseur de 1,5 à 5 nm. L'épaisseur de la couche de silice formée peut être notamment déterminée par XPS (*"X-Ray Photoelectron Spectroscopy"*).
(B) l'incorporation du support modifié obtenu dans l'étape précédente dans le milieu de synthèse d'une zéolithe, ce par quoi la zéolithe s'ancre progressivement sur le support au cours de sa formation.
   Dans ce cadre, il est préférable d'introduire le support modifié issu de l'étape (A) dans un milieu gélifié (gel) tel qu'obtenu lors de la préparation d'une zéolithe par voie hydrothermale. Dans ce cas, l'addition du support dans le milieu de synthèse de la zéolithe se fait de préférence immédiatement avant, pendant, ou immédiatement après un mûrissement du milieu de synthèse. Typiquement, le support modifié issu de l'étape (A) est ajouté au gel avant ou au cous du mûrissement et le mélange obtenu à l'issue du mûrissement est transvasé dans un récipient adapté aux synthèses par voie hydrothermale, par exemple dans un autoclave chemisé en Téflon. Dans ce cadre, on peut avantageusement employer la méthode décrite dans l'article de B. Louis et al. dans Applied Catalysis A210, p.103 (2001).
   Il est à souligner qu'on peut éventuellement répéter l'étape (B) pour effectuer le dépôt successif de plusieurs couches de zéolithe, le nombre de dépôts effectués permettant notamment de réguler l'épaisseur de la couche déposée.

Quel que soit le mode de préparation exact du catalyseur utilisé selon l'invention, le support de SiC utilisé pour sa constitution a préférentiellement une surface spécifique la plus élevée possible, et ce notamment de façon à assurer un ancrage le plus efficace possible de la zéolithe sur le support. Selon l'invention, on peut employer support de carbure de silicium ayant de préférence une surface spécifique d'au moins 10 m²/g, voire d'au moins 20 m²/g, cette surface spécifique restant en règle générale inférieure à 600 m²/g, le plus souvent inférieure à 400 m²/g, et typiquement inférieure à 200 m²/g, voire à 100 m²/g. Selon un mode de réalisation, la surface spécifique du support de carbure de silicium est comprise entre 5 et 25 m²/g. Au sens de la présente description, le terme de "surface spécifique" se réfère à la surface spécifique BET, telle que déterminée par adsorption d'azote, selon la méthode bien connue dite de BRUNAUER - EMMET - TELLER qui est décrite dans The journal of the American Chemical Society, volume 60, page 309 (1938), et correspondant à la norme internationale ISO 5794/1 (annexe D).

Les carbure de silicium de type β-SiC présentent, entre autres avantages, celui de posséder une porosité particulière, qui comprend essentiellement des macropores et des mésopores, avec des tailles de pores allant typiquement de 4 à 100 nm. Cette porosité est en outre sensiblement exempte de micropores qui seraient susceptibles d'engendrer des problèmes de diffusion des réactifs et des produits de réaction. Au sens de la présente description, on entend par "micropores" des pores de taille inférieure à 2 nm, les "macropores" désignant des pores de dimensions supérieures à 50 nm et les "mésopores" des pores de taille intermédiaire, allant de 2 et 50 nm. La porosité spécifique des carbures de silicium de type β-SiC permet une très bonne accessibilité du méthanol au niveau des sites catalytiques, ainsi que d'excellentes propriétés d'évacuation des produits formés. Ces échanges optimisés entre catalyseur et milieu extérieur se traduisent notamment en termes de rendement accru pour la réaction de conversion du méthanol en diméthyl éther. Par ailleurs, il semble pouvoir être avancé que ces échanges optimisés permettent d'inhiber les phénomènes d'empoisonnement du catalyseur par cokage (phénomène de déposition de carbone sur la catalyseur dû à des réactions secondaires). Un support de carbure de silicium de type β-SiC du type précité peut notamment être obtenu par réaction gaz/solide entre du SiO gazeux et du carbone solide. Dans ce cadre, on effectue en général la réaction en employant des vapeurs de SiO générés *in situ* dans le réacteur ou dans la matrice d'un solide précurseur. Un avantage des support à base de β-SiC est que leur synthèse conduit généralement directement à l'obtention de matériaux macroscopiques sans nécessiter d'étape de mise en forme supplémentaire. Ainsi, dans le cadre de la présente invention, on peut utiliser un support à base de β-SiC tel qu'obtenu directement après sa synthèse pour y effectuer un dépôt de la zéolithe. Selon un autre mode de réalisation, le support à base de β-SiC est prétraité thermiquement suite à sa synthèse, typiquement à une température de 900°C, le cas échéant de préférence pendant au moins une heure.

Le support de SiC des catalyseurs utiles selon l'invention peut être employé sous différentes formes.

Ainsi, selon un mode de réalisation envisageable, le support de SiC peut se présenter sous forme d'objets macroscopiques de type grains, extrudés, bâtonnets, monolithes, ou tubes ayant typiquement une surface spécifique comprise entre 5 et 50 m²/g, le plus souvent entre 5 et 30 m²/g. Dans ce cadre, on peut par exemple utiliser des supports de SiC du type de ceux obtenus selon les procédés décrits dans les documents EP 0 313 480, EP 0 440 569, US 5,217,930, EP 0511 919, ou EP 0 543 751 qui permettent d'accéder à des matériaux de tailles et de formes variées.

Selon un autre mode de réalisation particulièrement avantageux de l'invention, le support de SiC utilisé a une structure macroscopique ouverte. Dans ce cadre, le support de SiC a de préférence une structure de mousse alvéolaire rigide, ce support ayant alors avantageusement une surface spécifique comprise entre 5 et 400 m²/g, cette surface spécifique étant le plus souvent comprise entre 5 et 50 m²/g, avantageusement supérieure à 10 m²/g, et de préférence supérieure à 20 m²/g, plus avantageusement supérieure à 30 m²/g.

La surface spécifique d'un support de SiC structure macroscopique ouverte tel qu'une mousse alvéolaire rigide utile selon l'invention est typiquement comprise entre 5 et 100 m²/g, le plus souvent entre 5 et 50 m²/g. Des mousses alvéolaires de SiC de ce type peuvent notamment être obtenues selon le procédé décrit dans les documents EP 543 752, US 5,449,654 ou US 6,251,819. Des mousses rigides alvéolaire de β-SiC particulièrement bien adaptées selon l'invention présentent de préférence des ouvertures de pores comprises entre 300 et 5000 microns, avantageusement entre 1000 et 4000 microns, notamment entre 2000 et 3500 microns. La porosité ouverte (macroporosité) de la mousse rigide alvéolaire peut varier de 30 à 90%, de préférence 35 à 80%, notamment de 40 à 70% en volume par rapport au volume total du matériau.

L'emploi d'un support de SiC à structure de mousse alvéolaire rigide au lieu d'un support plus compact conduit à une amélioration encore plus nette de la stabilité du catalyseur, ainsi qu'à une amélioration significative de l'activité du catalyseur pour la réaction de conversion du méthanol en diméthyl éther.

De plus, les travaux des inventeurs ont permis d'établir que l'emploi de supports de type mousses alvéolaires du type précité induit des pertes de charges beaucoup plus limitées que dans le cas de matériaux plus compact.

En particulier, la structure poreuse très ouverte des mousses alvéolaire rigides de SiC permet de réduire d'une manière considérable les problèmes de perte de charge à travers le lit catalytique. Plus précisément, la structure alvéolaire permet d'utiliser des vitesses spatiales de réactifs (sous forme gazeux) beaucoup plus élevées sans souffrir de perte de charge excessive au travers du lit catalytique.

Il est également à noter que l'emploi d'un support de type mousse alvéolaire rigide permet une bonne diffusion du méthanol vers les sites catalytiques actifs (qui se trouvent sur la surface ou en partie dans la porosité du catalyseur) ainsi que l'évacuation du diméthyl éther produit hors de la zone catalytique, et ce même avec l'emploi des débits de réactifs élevés qui sont en général requis pour atteindre une productivité optimisée du procédé de conversion. Ces effets sont particulièrement intéressants dans le cas de réactions consécutives telles que mises en oeuvre dans le procédé de l'invention. Ce phénomène s'explique notamment par le fait que l'épaisseur des parois solides de la mousse rigide est relativement fine par rapport à celle d'un grain ou d'un extrudés, cette faible épaisseur permettant une meilleure diffusion des différentes espèces, et ce tout particulièrement lorsque la zéolithe est déposée sur ce support sous la forme d'une couche suffisamment fine.

De plus, les supports à porosité ouverte de type mousses alvéolaires rigide présentent une structure connexe qui leur confère de bonnes propriétés de conduction de la chaleur, améliorées par rapport à l'emploi d'un support plus compact tel qu'un support sous forme de grains, lesquels sont utilisés empilés les uns contre les autres au sein du réacteur avec des contacts grain-grain moins efficaces. En effet, dans le cas de l'emploi d'un support de SiC ayant une structure rigide de mousse alvéolaire le transfert de chaleur est de deux ordres, à savoir :
(i) un transfert de chaleur local, du site catalytique (la zéolithe déposée sur ledit support) vers le support de SiC et
(ii) un transfert de chaleur global, sur l'ensemble de la mousse interconnectée par des ponts rigides connexes.

L'existence de ce transfert de chaleur global, lié à la structure du support de porosité ouverte employé se révèle particulièrement pour permettre une évacuation de la forte chaleur de réaction dégagée par la réaction catalysée, et ce tout particulièrement lorsque l'on travaille à grande vitesses de réactifs en vue d'une augmentation de la productivité.

De par leur structure spécifique, les supports à porosité ouverte de type mousses alvéolaires rigide permettent de réduire de façon significative les phénomènes de désactivation du catalyseur observés avec les catalyseurs actuellement connus, et ce tout particulièrement lorsque la zéolithe est déposée sur ce support sous la forme d'une couche de faible épaisseur, avantageusement inférieure à 10 nm, et plus préférentiellement inférieure à 5 nm.

Les supports à porosité ouverte de type mousses alvéolaires rigide présentent en outre une résistance mécanique non négligeable, rendant possible des modes de chargement du réacteur différents que ceux utilisés avec des catalyseurs sous forme de grains ou d'extrudés. En particulier, il peut être envisagé d'employer les supports sous la forme de matériaux macroscopiques de grandes dimension capable d'épouser le volume interne du catalyseur, typiquement des mousses sous la forme de cylindres ayant un diamètre égal (ou légèrement inférieur) au diamètre interne d'un réacteur cylindrique, ou bien sous la forme de matériaux empilables ou juxtaposables pour occuper le volume un interne de réacteurs de grandes dimensions.

Au besoin, le catalyseur peut être activé préalablement à la réaction selon des méthodes connues, par exemple en traitant la zéolithe par un sel d'ammonium (par exemple le chlorure d'ammonium) puis en traitant thermiquement la zéolithe obtenue pour éliminer les espèces ammoniaquées.

Par ailleurs, le plus souvent, notamment pour faciliter la diffusion des réactifs et des produits au niveau du catalyseur, ainsi que pour faciliter les échanges thermiques locaux entre les sites catalytiques actifs et le support de SiC, il est préférable que la zéolithe immobilisée sur le support de SiC soit sous la forme d'une couche déposée d'épaisseur relativement faible, cette couche ayant de préférence une épaisseur moyenne inférieure à 50 microns, plus préférentiellement inférieure à 20 micron et plus avantageusement encore inférieure à 10 microns. Pour conserver une activité catalytique suffisante, il est toutefois préférable que cette épaisseur moyenne soit d'au moins 1 micron, plus préférentiellement d'au moins 3 microns. Des épaisseurs moyennes bien adaptés selon l'invention sont par exemple de l'ordre de 5 à 10 microns. L'épaisseur moyenne de la couche de zéolithe à laquelle il est fait référence ici peut être déterminée par microscopie électronique à balayage sur des échantillons en coupe polie.

La mise en oeuvre spécifique du catalyseur de l'invention permet de conduire la réaction de déshydratation catalytique du méthanol en diméthyl éther dans un large domaine de température sans perte d'activité du catalyseur, ce qui constitue encore un autre avantage des catalyseurs utilisés selon l'invention. Le plus souvent, il est préférable de conduire la réaction à une température comprise entre 200 et 500°C, de préférence à des températures inférieures à 400°C, par exemple entre 220 et 350°C, notamment entre 250 et 300°C.

Par ailleurs, avec les catalyseurs supportés employés selon l'invention, la réaction de déshydratation catalytique du méthanol en diméthyl éther n'a pas besoin d'être effectuée sous pression. Elle peut ainsi être effectuée sous pression atmosphérique, ce qui se traduit en terme de facilité de mise en oeuvre du procédé et de coûts réduits. De façon plus générale, la réaction de déshydratation catalytique du méthanol en diméthyl éther peut être effectuée sous des pressions supérieures ou égales à la pression atmosphérique, en particulier à des pressions moyennes généralement utilisées dans les unités de fabrication de méthanol ou en fonction des besoins post-réaction et aussi pour des raisons liées à la productivité du procédé. Le procédé peut ainsi être conduit à des pressions allant de 1 à 50 bar, par exemple à des pressions de 2 à 40 bar, en particulier de 5 à 30 bar.

Dans le cade de la présente invention, la réaction de déshydratation catalytique du méthanol peut typiquement être effectuée avec une vitesse spatiale horaire de 1 à 20 h⁻¹, par exemple de 1 à 5 h⁻¹, NTP.

Le procédé de l'invention permet de conduire très facilement la réaction de conversion du méthanol en diméthyl éther, et ce avec des taux conversion qui sont généralement élevés et qui, surtout, restent stables au cours du temps.

Par ailleurs, dans le procédé de l'invention, le méthanol mis en oeuvre dans la réaction de déshydratation catalytique n'a pas besoin d'être purifié. Il peut ainsi contenir un certain nombre d'impuretés ou de composés additionnels (tels que de l'eau par exemple), sous réserve naturellement que ces impuretés ne soient pas de nature à affecter la stabilité du catalyseur ou à conduire à des réactions secondaires indésirables dans le cadre de l'application visée.

Ainsi, selon un mode de réalisation de l'invention adapté à la synthèse de diméthyl éther relativement pur, on peut mettre en oeuvre un méthanol sensiblement exempt d'impuretés dans la réaction de déshydratation.

Selon un autre de réalisation adapté à la mise en oeuvre de l'invention, on peut également employer un méthanol brut dans la réaction de déshydratation (par exemple du méthanol brut issu de la biomasse) ou bien encore, de façon plus générale un milieu comprenant du méthanol en mélange avec d'autres composés et/ou impuretés, ce méthanol brut ou milieu à base de méthanol comprenant alors le plus souvent de 10 à 90% en masse de méthanol. Dans ce cadre, il se révèle le plus souvent avantageux que la teneur en méthanol soit d'au moins 20%, et plus préférentiellement d'au moins 30% en masse.

Différents aspects et avantages du procédé de l'invention apparaîtront de façon encore plus explicite à la lumière des exemples ci-après, donnés en référence aux Figures ci annexées, où :
- la Figure 1 montre l'évolution de la réaction de conversion de méthanol en diméthyl éther dans les conditions de l'exemple 3 ,
- la Figure 2 montre l'évolution de la réaction de conversion de méthanol en diméthyl éther dans les conditions de l'exemple 4
- la Figure 3 montre l'évolution de la réaction de conversion de méthanol en diméthyl éther dans les conditions de l'exemple 5
- La Figure 4 est un cliché en microscopie électronique en transmission du catalyseur C2 utilisé dans les exemples.

### EXEMPLE 1 :

### Préparation d'une zéolithe ZSM-5 supportée sur des extrudés de SiC (catalyseur C1)

Dans cet exemple, on a préparé un catalyseur C1 en effectuant le dépôt d'une couche de zéolithe sur un support de β-SiC à partir d'un gel précurseur, selon la voie hydrothermale. Plus précisément, on a mis en oeuvre les étapes ci-après :

### 1.1 Prétraitement du support de SiC

Dans cet exemple, on a utilisé à titre de support des extrudés de β-SiC de forme cylindrique ayant une longueur de 5 mm et un diamètre de 2 mm. La surface spécifique BET de ces extrudés de β-SiC est égale à 5 m²/g.

Ces extrudés de β-SiC ont été calcinés sous air à 900°C pendant 5 heures, de façon à former une couche de silice sur leur surface présentant une épaisseur de 5 à 10nm.

### 1.2 Préparation d'un gel de précurseur de zéolithe

Dans un bécher de 500 mL, à température ambiante (25°C), on a versé 200 mL d'eau distillée auxquels on a ensuite ajouté 2,23 g de chlorure de sodium.

Sous agitation vigoureuse, on a introduit 0,123 g d'aluminate de sodium anhydre (NaAlO₂), puis 24 mL d'hydroxyde de tétrapropylammonium (TPAOH, de formule ⁺N(C₃H₇)₄. OH⁻, assurant le rôle de structurant organique).

Toujours sous agitation, on a introduit 14 mL de tétraéthoxysilane (TEOS, de formule Si(OC₂H₅)₄ ). Cette addition a été fractionnée en sept additions de 2mL chacune, en laissant le milieu sous agitation quelques minutes entre chaque addition, de façon à s'assurer de la dissolution complète du TEOS.

On a ainsi obtenu un gel aqueux dont la composition molaire est la suivante :
TPAOH : TEOS : NaCl : NaAlOH : H₂O = 2,16 : 5,62 : 3,43 : 0,13 : 1000

### 1.3 Addition du SiC et mûrissement du milieu

Le β-SiC traité thermiquement tel qu'obtenu à l'issue de l'étape 1.1 a été introduit dans le gel obtenu dans l'étape 1.2,

On a laissé mûrir le milieu ainsi obtenu en le laissant sans agitation à température ambiante (25°C) pendant 4 heures.

### 1.4 Formation de la zéolithe sur le support β-SiC

Après mûrissement, le milieu a été transvasé dans un autoclave chemisé en Téflon, qu'on a placé en étuve à 170°C pendant 48 heures.

La synthèse de la zéolithe ainsi effectuée a été conduite dans des conditions autogènes.

Au bout des 48 heures de traitement thermique, l'autoclave a été refroidi et on a récupéré le solide présent dans le milieu. Ce solide est alors rincé par filtration puis subi un lavage abondant à l'eau distillée suivi d'une sonication de 30 minutes, et enfin un séchage à l'étuve.

On a ensuite traité thermiquement le solide obtenu à 500°C pendant 5 heures, de façon à éliminer le structurant organique.

### 1.5 Dépôt d'une seconde couche de zéolithe

Le matériau tel qu'obtenu à l'issu de l'étape 1.4 a été introduit dans un gel tel qu'obtenu dans l'étape 1.2, maintenu sous agitation.

On a laissé mûrir le milieu ainsi obtenu en le laissant sans agitation à température ambiante (25°C) pendant 4 heures.

Après mûrissement, le milieu a été transvasé dans un autoclave chemisé de Téflon, qu'on a placé en étuve à 170°C pendant 60 heures (conditions autogènes).

Au bout des 60 heures de synthèse, l'autoclave a été refroidi et on a récupéré le solide présent dans le milieu. Ce solide est alors rincé par filtration puis subi un lavage abondant à l'eau distillée et enfin un séchage à l'étuve. On a ensuite traité le solide obtenu à 500°C pendant 5 heures, de façon à éliminer le structurant organique.

### 1.6 Obtention de la forme définitive du catalyseur ("Activation")

Le matériau solide tel qu'obtenu à l'issue de l'étape 1.5 a été introduit dans une solution est mis en présence d'une solution de NH₄Cl à 1 M, et le mélange a été porté à reflux pendant 16 heures.

On a ainsi obtenu la forme ammonium de la zéolithe.

A l'issue de ce traitement, le solide présent dans le milieu a été filtré, lavé abondamment à l'eau distillée, puis séché et enfin calciné à 550°C pendant 5 heures. Ce dernier traitement thermique conduit à une élimination de l' ammoniaque, ce qui conduit à la forme acide de la zéolithe.

On a ainsi obtenu le catalyseur C1 (où la zéolithe est sous sa forme H-ZSM-5).

On a vérifié par analyse de diffraction des rayons X que le catalyseur C1 obtenu présente les raies de diffraction caractéristiques de la zéolithe H-ZSM-5, à savoir les raies caractéristiques de la structure cristalline MFI. L'indexation de ce pic a été faite en accord avec la banque de données du JCPDS (*Joint Commitee on Powder Diffraction Standards*). Ces raies sont également observées pour le solide obtenu à l'issue de l'étape 1.4, mais avec une intensité relative inférieure, ce qui indique que l'étape 1.5 a effectivement augmenté la quantité de zéolithe déposée sur le support.

Par ailleurs, des clichés de microscopie électronique indiquent que toute la surface du support de SiC est couverte de façon homogène par des cristaux de zéolithe ZSM-5, qui sont des cristaux sensiblement hexagonaux de dimension supérieure à 4 microns. Cette couverture homogène du support n'affecte pas la morphologie initiale du support.

La surface spécifique déterminée par adsorption d'azote du catalyseur C1 obtenu est de 55 m²/g, soit une surface onze fois plus importante que celle des extrudés seuls.

### EXEMPLE 2 :

### Préparation d'une zéolithe ZSM-5 supportée sur une mousse de SiC (catalyseur C2)

Dans cet exemple, on a préparé un catalyseur C2 en effectuant le dépôt d'une zéolithe sur un support de β-SiC à partir d'un gel précurseur, selon la voie hydrothermale, dans les mêmes conditions que dans les étapes 1.2 à 1.5 de l'exemple 1, mais en substituant les extrudés employés dans l'exemple 1 par une mousse alvéolaire de β-SiC de surface spécifique BET égale à 19 m²/g et ayant les caractéristiques suivantes : mousses alvéolaires obtenues par le procédé décrit dans le brevet FR2860992 présentant une ouverture de pores de 1750 microns, 25 mm de diamètre, et 4 cm de longueur.

Comme les extrudés de l'exemple 1, la mousse de β-SiC employée dans le présent exemple 2 a été préalablement calcinée sous air à 900°C pendant 5 heures, de façon à former une couche de silice sur sa surface.

On a ensuite préparé un gel précurseur dans les conditions de l'étape 1.2 de l'exemple 1, auquel on a ajouté la mousse de β-SiC prétraitée avant de laisser mûrir le milieu. On a ensuite formé la zéolithe sur le support dans les conditions de l'étape 1.4 de l'exemple 1, puis on a effectué le dépôt d'une seconde couche de zéolithe qu'on a ensuite mises sous forme acide, respectivement dans les conditions des étapes 1.5 et 1.6 de l'exemple 1.

A l'issue de ces différentes étapes, on a obtenu le catalyseur C2.

Là encore, des analyses par microscopie électronique indiquent que toute la surface du support de β-SiC est couverte de façon homogène par des cristaux de zéolithe ZSM-5, qui sont des cristaux sensiblement hexagonaux de dimension inférieure à 4 microns. Cette couverture homogène du support n'affecte pas la morphologie initiale du support comme l'illustre la Figure 4 ci-annexée.

Cette fois aussi, le dépôt de la zéolithe sur le support de mousse alvéolaire a permis d'obtenir une surface spécifique élevée, à savoir de 120 m²/g, soit une surface 6 fois supérieure à la mousse seule.

### EXEMPLE 3 :

### Synthèse de diméthyl éther utilisant catalyseurs C1 et C2

Chacun des catalyseurs C1 et C2 préparés dans les exemples 1 et 2 a été utilisé pour catalyser la réaction de conversion du méthanol en diméthyl éther.

Dans tous les cas, la réaction de conversion a été conduite sous pression atmosphérique, à une température de 400°C.

Le méthanol a été introduit à l'aide d'une pompe HPLC avec un débit de 0,5 mL/min dans un flux d'argon (Argon 4.5 commercialisé par Air Liquide) où il est vaporisé et entraîné vers un réacteur comprenant le catalyseur. Le débit du flux d'argon est de 80 cc/min.

Le réacteur qui a été utilisé est constitué d'un tube en quartz de diamètre interne égal à 1 pouce (25,4 mm) et muni d'un fritté sur lequel on a déposé 4 g du catalyseur considéré. Ce réacteur est placé dans un four porté à 400°C.

La température a été contrôlée à l'aide de deux thermocouples, l'un étant placé dans le four à l'extérieur du réacteur pour réguler la température du four et l'autre à l'intérieur du lit catalytique. Par ailleurs, toutes les lignes en aval du réacteur ont été chauffées à l'aide de fil chauffant les maintenant à 100°C, de façon à éviter une condensation des produits de la réaction.

Les produits formés ont été analysés par chromatographie en phase gazeuse pour les produits gazeux, et en phase liquide pour les produis liquides, à l'aide d'un chromatographe Varian CP 3800 équipé d'une colonne capillaire DB-1 (longueur : 30 m - diamètre interne : 0,53 mm) et d'un détecteur FID (détecteur à ionisation de flamme). L'acquisition des données a été effectuée de façon automatisée à l'aide du logiciel fourni par la société Varian, ce qui permet de contrôler le programme de température et de traiter le signal envoyer par le détecteur (intégration des pics).

A des fins de comparaison, la réaction a été conduite dans les mêmes conditions avec deux autres catalyseurs témoins à savoir :
T1 : zéolithe ZSM-5 non supportée, préparée dans les conditions de l'étape 1.4 de l'exemple 1, mais sans addition préalable de support β-SiC
T2 : catalyseur commercial Zeolyst (CBV 8014) (zéolithe de type MFI caractérisée par un rapport Si/Al de 40)

La Figure 1 ci-jointe montre l'évolution du rendement en diméthyl éther au cours du temps avec les quatre catalyseurs testés.

Avec les catalyseurs C1 et C2 selon l'invention, le rendement se stabilise rapidement et reste sensiblement constant jusqu'à la fin des 17 heures de réaction.

Pour le catalyseur C1, le rendement reste supérieur à 50% une fois stabilisé et ce tout au long de la réaction.

Des résultats encore plus intéressants sont obtenus pour le catalyseur C2 où la conversion et le rendement en DME restent stable entre 80 et 90 % après stabilisation, et ce tout au long de la réaction. L'amélioration observée par rapport au catalyseur C1 s'explique vraisemblablement au moins en partie par la capacité accrue que possède la structure de mousse alvéolaire pour évacuer la chaleur de réaction.

A l'inverse, avec les témoins T1 et T2, on observe une perte d'activité drastique dès les premières heures de réaction.

Les résultats obtenus en ce qui concerne la sélectivité montrent l'absence de formation d'hydrocarbures saturés résultant de la désactivation du catalyseur, ce qui confirme la remarquable stabilité des catalyseurs selon l'invention, en particulier celle du catalyseur C2 qui permet de convertir 84% du méthanol introduit dans le réacteur, en le transformant à 95% en diméthyl éther.

### EXEMPLE 4 :

### Synthèse de diméthyl éther utilisant le catalyseur C2

Le catalyseur C2 préparé dans l'exemple 2 a été utilisé pour catalyser la réaction de conversion du méthanol en diméthyl éther dans des conditions relativement similaires à celles de l'exemple 3, à savoir sous pression atmosphérique, mais cette fois-ci à une température de 270°C.

Le méthanol a été introduit à l'aide d'une pompe HPLC en utilisant deux débits 0,6 et 1,4 mL/min dans un flux d'argon (Argon 4.5 commercialisé par Air Liquide) où il est vaporisé et entraîné vers un réacteur comprenant le catalyseur. Deux valeurs pour le flux d'argon ont été prises pendant le test : 80 et 160 mL/min.

Le réacteur qui a été utilisé est constitué d'un tube en quartz de diamètre interne égal à 1 pouce (25,4 mm) et muni d'un fritté sur lequel on a déposé 4 g du catalyseur considéré. Ce réacteur est placé dans un four porté à 270°C.

La température a été contrôlée à l'aide de deux thermocouples, l'un étant placé dans le four à l'extérieur du réacteur pour réguler la température du four et l'autre à l'intérieur du lit catalytique. Par ailleurs, toutes les lignes en aval du réacteur ont été chauffées à l'aide de fil chauffant les maintenant à 100°C, de façon à éviter une condensation des produits de la réaction.

Les produits formés ont été analysés par chromatographie en phase gazeuse pour les produits gazeux, et en phase liquide pour les produis liquides, à l'aide d'un chromatographe Varian CP 3800 équipé d'une colonne capillaire DB-1 (longueur : 30 m - diamètre interne : 0,53 mm) et d'un détecteur FID (détecteur à ionisation de flamme). L'acquisition des données a été effectuée de façon automatisée à l'aide du logiciel fourni par la société Varian, ce qui permet de contrôler le programme de température et de traiter le signal envoyé par le détecteur (intégration des pics).

La Figure 2 ci-annexée présente l'évolution du rendement en DME en fonction du temps et des conditions de réactions.

L'augmentation de la quantité de méthanol ne change pas le comportement de catalyseur et le rendement en DME reste stable, autour de 0,8.

### EXEMPLE 5 :

### Synthèse de diméthyl éther utilisant le catalyseur C2

Le catalyseur C2 préparé dans l'exemple 2 a été utilisé pour catalyser la réaction de conversion du méthanol en diméthyl éther dans des conditions relativement similaires à celles de l'exemple 3, à savoir sous pression atmosphérique, mais cette fois-ci à une température de 270°C.

Le méthanol a été introduit à l'aide d'une pompe HPLC en utilisant un seul débit 0,6 dans un flux d'argon (Argon 4.5 commercialisé par Air Liquide) où il est vaporisé et entraîné vers un réacteur comprenant le catalyseur. Une valeur pour le flux d'argon a été prise pendant le test : 80 ml/min.

Le réacteur qui a été utilisé est constitué d'un tube en quartz de diamètre interne égal à 1 pouce (25,4 mm) et muni d'un fritté sur lequel on a déposé 4 g du catalyseur considéré. Ce réacteur est placé dans un four porté à 270°C.

La température a été contrôlée à l'aide de deux thermocouples, l'un étant placé dans le four à l'extérieur du réacteur pour réguler la température du four et l'autre à l'intérieur du lit catalytique. Par ailleurs, toutes les lignes en aval du réacteur ont été chauffées à l'aide de fil chauffant les maintenant à 100°C, de façon à éviter une condensation des produits de la réaction.

Les produits formés ont été analysés par chromatographie en phase gazeuse pour les produits gazeux, et en phase liquide pour les produis liquides, à l'aide d'un chromatographe Varian CP 3800 équipé d'une colonne capillaire DB-1 (longueur : 30 m - diamètre interne : 0,53 mm) et d'un détecteur FID (détecteur à ionisation de flamme). L'acquisition des données a été effectuée de façon automatisée à l'aide du logiciel fourni par la société Varian, ce qui permet de contrôler le programme de température et de traiter le signal envoyé par le détecteur (intégration des pics).

Le catalyseur C2 a été testé avec le méthanol pur et par comparaison avec du méthanol « crude », à savoir un mélange CH₃OH/H₂O = 80/20 % vol.

La Figure 3 jointe en annexe montre l'évolution du rendement en diméthyl éther au cours du temps.

Au début le composite montre un meilleur rendement dans la réaction avec du méthanol pur d'environ 0,8. Dans la réaction avec le méthanol brut, le composite présente une plus basse conversion mais une meilleure sélectivité et au bout de 18 heures le rendement est identique pour les deux réactions.

## Revendications

1. Procédé de préparation de diméthyl éther par déshydratation catalytique du méthanol, dans lequel on emploie, à titre de catalyseur, une zéolithe H-ZSM-5 immobilisée sur un support de carbure de silicium β-SiC, où le support de carbure de silicium a une surface spécifique supérieure ou égale à 5 m²/g.

2. Procédé selon la revendication 1, où le support de carbure de silicium du catalyseur est sous forme d'objets macroscopiques de dimensions supérieures à 1 mm, de type grains, extrudés, bâtonnets, monolithes, tubes, trilobés ou anneaux.

3. Procédé selon la revendication 1 ou 2, où le support de carbure de silicium du catalyseur est une mousse alvéolaire rigide, qui a des ouvertures de pores préférablement comprises entre 300 et 5000 microns, et de préférence une surface spécifique comprise entre 5 et 50 m²/g.

4. Procédé selon l'une quelconque des revendications 1 à 3, où la zéolithe immobilisée sur le support de SiC est sous la forme d'une couche d'épaisseur moyenne comprise entre 1 et 50 microns, de préférence entre 3 et 20 microns.

5. Procédé selon l'une quelconque des revendications 1 à 4, où la réaction de déshydratation catalytique du méthanol en diméthyl éther est conduite à une température comprise entre 200 et 500°C .

6. Procédé selon l'une quelconque des revendications 1 à 5, où la réaction de déshydratation catalytique du méthanol en diméthyl éther est effectuée sous une pression allant de 1 à 50 bar.

7. Procédé selon l'une quelconque des revendications 1 à 6, où la réaction de déshydratation catalytique du méthanol en diméthyl éther est effectuée sous pression atmosphérique.

8. Procédé selon l'une quelconque des revendications 1 à 7, où la réaction de déshydratation catalytique du méthanol est effectuée avec une vitesse spatiale horaire de 1 à 20 h⁻¹ NTP.

## Patentansprüche

1. Verfahren für die Herstellung von Dimethylether durch katalytische Dehydrierung des Methanols, wobei als Katalysator ein auf einem Träger aus Siliciumcarbid β-SiC immobilisiertes Zeolith H-ZSM-5 verwendet wird, wobei der Siliciumcarbidträger eine spezifische Fläche größer oder gleich 5 m²/g hat.

2. Verfahren nach Anspruch 1, wobei der Siliciumcarbidträger des Katalysators die Form makromikroskopischer Objekte hat, die größer als 1 mm sind, vom Typ Körner, Extruder, Stäbe, Monolithe, Rohre, Dreipässe oder Ringe.

3. Verfahren nach Anspruch 1 oder 2, wobei der Siliciumcarbidträger des Katalysators ein starrer zelliger Schaumstoff ist, der Porenöffnungen vorzugsweise zwischen 300 und 5000 Mikron inklusive und vorzugsweise eine spezifische Fläche zwischen 5 und 50 m²/g inklusive hat.

4. Verfahren nach einem der Ansprüche 1 bis 3, wobei der auf dem SiC-Träger immobilisierte Zeolith die Form einer Schicht mit einer mittleren Dicke zwischen 1 und 50 Mikron, vorzugsweise zwischen 3 und 20 Mikron, inklusive hat.

5. Verfahren nach einem der Ansprüche 1 bis 4, wobei die katalytische Dehydrierungsreaktion des Methanols in Dimethylether bei einer Temperatur zwischen 200 und 500 °C inklusive durchgeführt wird.

6. Verfahren nach einem der Ansprüche 1 bis 5, wobei die katalytische Dehydrierungsreaktion des Methanols in Dimethylether bei einem Druck von 1 bis 50 bar durchgeführt wird.

7. Verfahren nach einem der Ansprüche 1 bis 6, wobei die katalytische Dehydrierungsreaktion des Methanols in Dimethylether unter atmosphärischem Druck durchgeführt wird.

8. Verfahren nach einem der Ansprüche 1 bis 7, wobei die katalytische Dehydrierungsreaktion des Methanols in Dimethylether mit einer Raumgeschwindigkeit pro Stunde von 1 bis 20 h⁻¹ NTP durchgeführt wird.

## Claims

1. Process for the preparation of dimethyl ether by catalytic dehydration of methanol, wherein the used catalyst is a H-ZSM-5 zeolite immobilised on a β-SiC silicon carbide support, wherein the silicon carbide support has a specific surface area greater than or equal to 5 m²/g.

2. Process according to claim 1, wherein the silicon carbide support of the catalyst is in the form of macroscopic objects having dimensions greater than 1 mm, of the grain, extrudate, rod, monolith, tube, trilobe or ring type.

3. Process according to claim 1 or 2, wherein the silicon carbide support of the catalyst is a rigid cellular foam, with pore sizes preferably of from 300 to 5000 microns, and preferably a specific surface area of from 5 to 50 m²/g.

4. Process according to any one of claims 1 to 3, wherein the zeolite immobilised on the SiC support is in the form of a layer having an average thickness of from 1 to 50 microns, preferably from 3 to 20 microns.

5. Process according to any one of claims 1 to 4, wherein the catalytic dehydration reaction of methanol to dimethyl ether is conducted at a temperature of from 200 to 500°C.

6. Process according to any one of claims 1 to 5, wherein the catalytic dehydration reaction of methanol to dimethyl ether is carried out under a pressure ranging from 1 to 50 bar.

7. Process according to any one of claims 1 to 6, wherein the catalytic dehydration reaction of methanol to dimethyl ether is carried out under atmospheric pressure.

8. Process according to any one of claims 1 to 7, wherein the catalytic dehydration reaction of methanol is carried out with an hourly space velocity of from 1 to 20 h⁻¹ NTP.
